# FASCICULE DE BREVET EUROPEEN

(11) **EP 0 991 360 B1**
(45) Date de publication et mention de la délivrance du brevet: **31.03.2004**
(21) Numéro de dépôt: 98921540.5
(22) Date de dépôt: 15.04.1998
(51) Int. Cl.: A61B 17/16

(54) **FORET CHIRURGICAL RECUPERATEUR D'OS COMPORTANT UNE BUTEE A REPERE EN COULEUR**
CHIRURGISCHE BOHRMASCHINE MIT AUFSAMMELVORRICHTUNG FÜR KNOCHENSUBSTANZ
BONE RECOVERING SURGICAL DRILL COMPRISING A COLOURED MARK

(30) Priorité: 30.04.1997 FR 9705387
(43) Date de publication de la demande: 12.04.2000
(73) Titulaire: Implants Diffusion, 75020 Paris (FR)
(72) Inventeur: BOUKHRIS, Gilles, F-75012 Paris (FR)
(86) Numéro de dépôt international: PCT/FR1998/000760
(87) Numéro de publication internationale: WO 1998/048708

(56) Documents cités:
- EP-A- 0 515 274
- WO-A-93/24061
- WO-A-96/12453
- US-A- 4 710 075
- US-A- 5 261 818
- US-A- 5 575 650

## Description

La présente invention concerne un foret chirurgical récupérateur d'os avec une butée pour en limiter l'enfoncement et présentant une ou plusieurs gorges circulaires profondes entre la partie active du foret et la butée afin de retenir l'os de forage, selon le préambule de la revendication 1, un tel foret est connu du document US-A-4710075. L'invention propose un foret comportant de plus les caractéristiques de la seconde partie de la revendication 1. Cet os pourra dans un deuxième temps être récupéré à des fins de greffe osseuse. Ce foret chirurgical récupérateur d'os peut présenter cinq points spécifiques :
a) Une butée (B) fixe d'un diamètre supérieur à la partie active, servant à limiter l'enfoncement du foret et de toit à la zone de récupération d'os.
b) Un repère (d) en couleur étalé sur la butée afin de connaître la distance entre la pointe et la butée.
c) Un repère (e) sur la partie lisse non active (E) du foret afin de reconnaître le diamètre du foret.
d) Un ou plusieurs espaces circulaires (f) entre la pointe du foret et la butée ayant pour rôle de récupérer l'os du forage. Cet espace pourra avoir de un à plusieurs mm de largeur et cette gorge circulaire devra avoir une profondeur de un à plusieurs mm compatible avec la résistance du foret.
e) La partie active (A) se termine par un angle (h) supérieur à 90 degrés afin de faciliter le retrait de l'instrument en rotation.

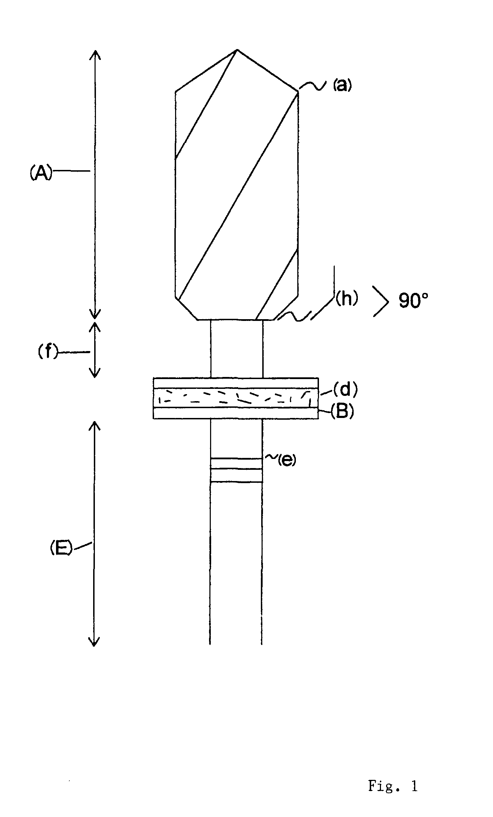

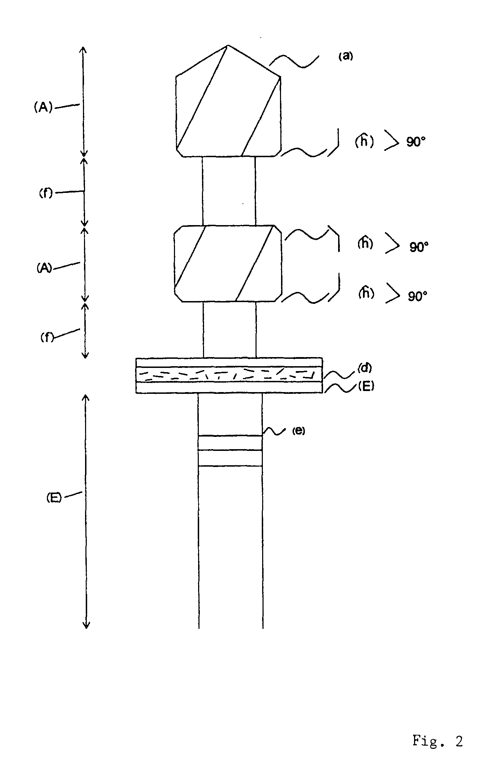

## Revendications

1. Foret chirurgical comportant une partie active (A) et une partie non active (E) ayant entre la partie active et non active une ou plusieurs gorges circulaires (f) de profondeur et de largueur suffisante pour récupérer l'os de forage ; ainsi qu'une butée d'un diamètre supérieur à la partie active (A) dont le but est de limiter l'enfoncement du foret **caractérisé en ce que** la butée du foret est une butée fixe avec un repère en couleur étalé sur cette dernière.

2. Foret chirurgical récupérateur d'os selon la revendication 1, **caractérisé en ce que** la ou les gorges circulaires (f) ont une profondeur de 1 à plusieurs mm et une largeur de 1 à plusieurs mm.

3. Foret chirurgical récupérateur d'os selon la revendication 1, **caractérisé en ce qu'**il présente sur sa partie non active (E) un signe indiquant le diamètre du foret (e).

## Claims

1. The surgical drill comprising an active part (A) and a nonactive part (E), having between the active and nonactive part one or more circular throats (f) of depth and sufficient width to recover the bone of drilling; as well as a thrust of a diameter higher than the active part (A) of which the goal is to limit the depression of the drill **characterized in that** the thrust of the drill is a dead stop with a reference mark color spread out over the latter.

2. Surgical drill recuperator of bone according to claim 1, **characterized in that** circular throats (f)ont a depth of 1 with several millimetres.

3. Surgical drill recuperator of bone according to claim 1, **characterized in that** it presents on its part nonactive (E) a sign indicating the diameter of the drill (e).

## Patentansprüche

1. Das chirurgische Bohrgerät, das ein aktives Teil (A) und ein nicht-aktives Teil (E), habend zwischen dem aktiven und nicht-aktiven Teil eine oder mehr kreisförmigen Kehlen (f) Tiefe und genügende Breite, den Knochen der Bohrung zurückzugewinnen enthält; sowie einen Schub eines Durchmessers stark als das aktive Teil (A), von dem das Ziel, den Tiefstand des **dadurch gekennzeichnete**n Bohrgeräts zu begrenzen ist, daß der Schub des Bohrgeräts ein toter Anschlag mit einer Bezugsmarkenfarbe ist, verbreiten Sie heraus über der letzten.

2. Chirurgischer Bohrgerätwärmeaustauscher des Knochens entsprechend Anspruch 1, in dem kreisförmige Kehlen gekennzeichnet (f) ont eine Tiefe von 1 mit einigen mm.

3. Chirurgischer Bohrgerätwärmeaustauscher des Knochens entsprechend Anspruch 1, **dadurch gekennzeichnet, daß** er auf seinem Teil darstellt, das (E) ein Zeichen nicht-aktiv ist, das den Durchmesser des Bohrgeräts anzeigt (e).
